(19) Europäisches Patentamt
European Patent Office
Office européen des brevets

(11) EP 1 121 857 B1

(12) # EUROPEAN PATENT SPECIFICATION

(45) Date of publication and mention of the grant of the patent:
**04.05.2005 Bulletin 2005/18**

(51) Int Cl.7: **A01N 33/12**, B27K 3/34, C07C 211/63, C01C 1/28, C01C 1/26

(21) Application number: **01101121.0**

(22) Date of filing: **09.06.1994**

(54) **Preparation of quarternary ammonium carbonates**

Herstellung quaternärer Ammonium-Carbonate

Préparation des carbonates d'ammonium quaternaire

(84) Designated Contracting States:
**AT BE CH DE DK ES FR GB GR IE IT LI LU MC NL PT SE**
Designated Extension States:
**SI**

(30) Priority: **09.06.1993 US 74136**
**09.06.1993 US 74312**
**09.06.1993 US 74313**
**09.06.1993 US 74314**

(43) Date of publication of application:
**08.08.2001 Bulletin 2001/32**

(62) Document number(s) of the earlier application(s) in accordance with Art. 76 EPC:
**94920194.1 / 0 702 517**

(73) Proprietor: **LONZA INC.**
**Fair Lawn New Jersey 07410 (US)**

(72) Inventor: **Walker, Leigh**
**Macungie, PA 18062 (US)**

(74) Representative: **Riegler, Norbert Hermann**
**Lonza AG**
**Patentabteilung**
**Postfach**
**4002 Basel (CH)**

(56) References cited:
**US-A- 4 857 525** **US-A- 5 354 434**

- **E MÜLLER (ED ): "Methoden der organischen Chemie (Houben-Weyl), Vol. XI/2. Stickstoffverbindungen II und III" , METHODEN DER ORGANISCHEN CHEMIE,DE,STUTTGART, G. THIEME VERLAG, VOL. BAND 11, PAGE(S) 620-626 XP002084862 * page 624, paragraph 3 - page 636, paragraph 1 ***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

This invention relates to the indirect synthesis of $C_1$-$C_{20}$ alkyl or aryl-substituted alkyl, $C_8$-$C_{20}$ alkyl quaternary ammonium carbonate compositions (carbonate quats) from corresponding quaternary ammonium chlorides. Di $C_8$-$C_{12}$ alkyl quaternary carbonate compositions are particularly useful in wood preservative systems, as surfactants and as biocides.

### BACKGROUND OF THE INVENTION

Quaternary ammonium compounds (quats), and particularly didecyldimethylammonium chloride (DDAC).

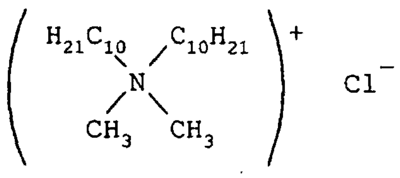

(I)

are commonly used as wood preservatives because they possess resistance properties to fungi and termites, to loss of strength, and to electrical sensitivity similar to those of commonly used acidic copper/chromium/arsenic solution (CCA) or ammoniacal copper and arsenic salt solution preservatives. See Proc. of the Am.Wood Pros. Assoc. 80: 191-210 (1984). Although chloride quats do not include potentially dangerous heavy metals, didecyldimethylammonium chloride leaches rapidly in soil (Nicholas et al., Forest Prod.J.41:41 (1991), and therefore, does require coupling with copper salt.

Findlay et al., U.S. Patent No. 4,929,454, disclose a method of preserving wood by impregnation with a quaternary ammonium compound and at least one of zinc and copper, wherein the quat anion is chosen from the group consisting of hydroxide, chloride, bromide, nitrate, bisulfate, acetate, bicarbonate, and carbonate, formate, borate and fatty acids. These quats have distinct environmental and safety advantages over commonly used acidic copper/chromium/arsenic solution (CCA) or ammoniacal copper and arsenic salt solution preservatives in that potentially dangerous heavy metals are not included. The Findlay et al. quats require copper or zinc in order to render them relatively insoluble and to prevent them from leaching out of a treated substrate. The use of copper or zinc in the above formulations may yet raise environmental and corrosion concerns.

Additionally, didecyldimethylammonium chloride tends to absorb preferentially to the surface of the wood and does not uniformly treat the whole substrate. Finally, DDAC treated wood shows a surface erosion or ages upon exposure to light. See Preston et al., Proc. Am. Wood Pres. Assoc., 83:331 (1987).

The biocidal activities of various chloride quats against bacteria, fungi, and algae are tabulated in Cationic Surfactants, E. Jungerman Ed., pp. 56-57, Marcel Dekker, Inc., 1969. Nicholas, "Interaction of Preservatives with Wood," Chemistry of Solid Wood, Advances in Chemistry Series #207, Powell ed., (A.C.S. 1984) , notes that didecyldimethylammonium compounds and particularly DDAC are potential biocides. Preston, J.A.O.C.S. 60:567 (1983), concurs and suggests that maximum fungitoxicity is exhibited with dialkyldimethyl compounds having $C_{10}$-$C_{12}$ alkyl groups. Butcher et al., Chem.Abstracts No. 91:152627b, suggest that the presence of an acid or a base can affect the activity of didecyldimethylammonium quats.

Didecyldimethylammonium acetate was used as a phase transfer catalyst for an oxidation in Chem.Abstracts No. 97:9175. A wood preservative was prepared by autoclaving didecylmethylamine with gluconic acid and ethylene oxide in isopropanol to yield $[(C_{10}H_{21})_2CH_3N((CH_2)_2O)_nH]^+$ gluconate in Chem. Abstracts No. 109:124403x, while disinfectant solutions were prepared by exchanging a benzylammonium chloride with a chlorhexidine gluconate in Chem. Abstracts No. 103:109954f.

Microbiocidal compositions which include quaternary ammonium compounds of the formula $R^1N^+R^2R^3R^4$ $X^-$, wherein at least one of $R^1$, $R^2$, or $R^3$ is a $C_8$-$C_{30}$ alkyl or alkenyl group and the remainder of $R^1$, $R^2$ or $R^3$ is methyl, ethyl, $CH_2Ph$ or 4-pyridyl-methyl; $R^4$ is methyl or ethyl; and X is an anion of an acid having a $C_7$ or greater hydrophobic group, were disclosed in Chem. Abstracts Nos. 113:154360f and 113:153776j. Chem. Abstracts No. 112:79768u discloses compounds of the formula $R^1R^2R^3R^4N^+X^-$, wherein $R^1$, $R^2$, and $R^3$ are methyl, ethyl, benzyl, 4-pyridinomethyl and at least one is $C_8$-$C_{30}$ alkyl or alkenyl; $R^4$ is methyl or ethyl; and X is a counter anion of acids having $C_7$ or greater hydrophobic groups. Dimethyldidecylammonium dodecylbenzenesulfonate was demonstrated to impart long term rot resistance to wood without causing rust, while the chloride salts of similar compounds were demonstrated to cause rust.

Patton et al., U.S. Patent No. 5,004,760, disclose polymeric foams incorporating various dialkyldimethylammonium carboxylates such as didecyldimethylammonium poly(ethylene/acetate) and the like.

Quaternary ammonium compounds (quats) are typically prepared by the reaction:

$$R^1R^2R^3N + R^4X \rightarrow R^1R^2R^3R^4NX \quad \text{(II)}$$

wherein X is a halogen, a sulfate, a sulfo compound, or the like. When at least one of $R^1$, $R^2$, $R^3$, or $R^4$ is $C_{12}$ or longer, the product is an inert soap. Many of the inert soaps have biocidal activity against bacteria, fungi, algae, and related organisms.

Reaction (II) above is limited by the reactant $R^4X$ because $R^4$ must react with tertiary amines. For example, methyl chloride ($R^4X = CH_3Cl$) will react with a tertiary amine at less than 100°C to yield a quaternary compound $R_3N^+CH_3\ Cl^-$, while methanol or methyl acetate :$R^4X=CH_3OH$ or $CH_3COOCH_3$) will not, under similar reaction conditions.

General quaternary ammonium compounds with a sulfo group are easily prepared either by the reaction of a sulfate compound with a tertiary amine (III) or by a double exchange (IV).

$$R_3N + RSO_3CH_3 \rightarrow R_3NCH_3^+RSO_3^- \quad \text{(III)}$$

$$R_3N^+\ CH_3\ Cl^- + RSO_3^-\ Na^+ \rightarrow R_3NCH_3^+\ RSO_3^- + NaCl \quad \text{(IV)}$$

If trimethylamine is heated with carbon dioxide and methanol above 200°C and at 85 to 95 atmospheres, the carbonate quat, bis-tetrarnethylammonium carbonate, is prepared. Industrial Organic Nitrogen Compounds, Astle Ed. p 66, Reinhold Inc, 1961. However, this reaction is limited to the methyl compound because higher homologs decompose to olefins by the Hofman elimination reaction. See, Organic Reactions, 11, Chptr. 5, 377, Krieger Publishing Co., 1975.

Chem.Abstracts 110:212114 (1989) suggests that dimethyl carbonate will react with triethylamine in methanol in twelve hours at 115°C and under pressure to yield a methyl carbonate ester quat.

Chem.Abstracts 114:24824 (1991) discloses that 6-hydroxy-hexyldimethylamine reacts with dimethyl carbonate to yield a carbonate ester quat.

Quaternary ammonium hydroxides (hydroxy quats), an intermediate in the reaction scheme of the present invention, are currently prepared by the reaction of quaternary ammonium iodide with silver hydroxide (V).

$$RN^+(CH_3)_3\ I^- + AgOH \rightarrow RN^+(CH_3)_3OH^- + AgI \quad \text{(V)}$$

However, this reaction is costly, and it is difficult to recover the silver reagent. See, Organic Reactions, 11:Chptr 5, pp. 376-377, Krieger Publishing Co., 1975.

In an olefin synthesis, it has been suggested to treat a quaternary salt with aqueous sodium or potassium hydroxide followed by pyrolysis in order to form the hydroxy quat and then to decompose the hydroxy quat directly. However, in this method the hydroxy quat is not isolated and the conditions for its preparation are undesirable. See, Organic Reactions, 11:Chptr 5, pp. 376-377, Krieger Publishing Co., 1975.

Talmon et al., Science, 221, 1047 (1983), have used an ion exchange resin to convert didecyldimethylammonium bromide to didecyldimethylammonium hydroxide (VI).

$$(C_{12}H_{25})_2(CH_3)_2N^-\ Br + \text{Ion Exchange Resin} \rightarrow$$

$$(C_{12}H_{25})_2(CH_3)_2N^+OH^- \quad \text{(VI)}$$

However, 50 ml of ion exchange resin and two treatment steps were required to convert 3 grams of quaternary ammonium chloride to the corresponding hydroxide. Talmon et al. state that the hydroxy quat can be reacted with acids to make quats with different anions, and they have prepared didodecyldimethylammonium (DDDA) acetate, DDDA-formate, DDDA-propionate, DDDA-butyrate, DDDA-oxalate, DDDA-acrylate, DDDA-tartrate, DDDA-benzoate, and DDDA-octanoate. See also, Organic Synthesis, Collective Volume VI, 552, John Wiley Inc., 1988; Brady et al., J. Am.

Chem. Soc., 106:4280-4282, 1984; Brady et al., J. Phys. Chem., 90:9, 1853-1859, 1986; Miller et al., J. Phys. Chem, 91:1, 323-325, 1989; Radlinske et al., Colloids and Surfaces, 46:213-230, 1990.

Distearyldimethylammonium gluconate was prepared via ion exchange and subsequent reaction with an organic acid in Chem Abstracts No. 75:11917OU. Miller et al, Langmuir, 4:1363 (1988) prepared ditetradecyldimethylammonium acetate by ion exchange from a bromide.

Alternatively, quaternary ammonium hydroxide compositions have been prepared by treating a haloquat in an electrochemical cell with special cation exchange diaphragms between the cells. The hydroxy quat collects at one electrode, and the halide collects at the other. Hydroxy quats, $R^1R^2R^3R^4N^+OH^-$, wherein the R groups were $C_1$-$C_4$, were treated with carboxylic acids to make asymmetric quats that were used as capacitor driving electrolytes. See, Japanese Patent Publication No. 02-106,915 and Awata et al., Chemistry, Letters, 371 (1985). Awata et al. placed carboxylic acids in the cathode cell to react with tetraethylammonium hydroxide as it was formed.

Japanese Patent Publication No. 01-172,363 discloses the preparation of relatively low yields of tetraethylammonium hydroxide by reacting triethylamine with diethyl sulfate, heating the resultant quat with sulfuric acid to yield the sulfate quat, and reacting the sulfate quat with barium hydroxide to yield the short chain quat, tetraethylammonium hydroxide, and barium sulfate.

Di $C_8$-$C_{12}$ alkyl quaternary ammonium hydroxides prepared by ion exchange were used as strong bases to digest animal tissue by Bush et al., French Patent Publication No. 1,518,427.

Akzo discloses that the addition of a metallic hydroxide to a quaternary ammonium chloride such as didecyldimethylammonium chloride, in an aqueous medium, results in an equilibrium mixture of quaternary ammonium chloride and quaternary ammonium hydroxide (VII). This reaction can be driven to the right by the use of isopropanol as a solvent.

$$(R_4N)Cl + KOH \leftrightarrow (R_4N)OH + KCl \qquad \text{(VII)}$$

Akzo further discloses that the addition of a soap to a quaternary ammonium chloride yields a quaternary ammonium carboxylate (VIII).

$$(R_4N)Cl + R^1COONa \rightarrow (R_4N)\ (OOCR^1) + NaCl \qquad \text{(VIII)}$$

Jordan et al., U.S. Patent No. 3,281,458, disclose the preparation of dioctadecyldimethylammonium humate, ditallowdimethylammonium humate, dipentadecyldimethylammonium humate, and didodecyldimethylammonium humate by reacting humic acid, lignite, aqueous sodium hydroxide and a chloride quat.

Finally, Nakama et al., J.A.C.O.S., 67:717 (1990) report the interaction between anionic and cationic surfactant and particularly sodium laureate and stearyltrimethylammonium chloride, while Linderborg, U.S. Patent No. 4,585,795, disclose the use of synergistic mixtures of the alkali metal salt of certain biocidal organic acids, quaternary ammonium chlorides, and alkyl-pyridinium chlorides as control agents for short-term protection of timber against sapstain fungi and mildew.

Consequently, efforts have been directed to develop a safe, efficient and expedient method to prepare quaternary ammonium compounds that do not require potentially hazardous metal additives to treat wooden substrates effectively.

It has been discovered that useful $C_1$-$C_{20}$ alkyl or aryl-substituted alkyl, $C_8$-$C_{20}$ alkyl quaternary ammonium carbonates can be prepared, particularly by indirect synthesis from $C_1$-$C_{20}$ alkyl or aryl-substituted alkyl, $C_8$-$C_{20}$ alkyl quaternary ammonium chlorides, through $C_1$-$C_{20}$ alkyl or aryl-substituted alkyl, $C_8$-$C_{20}$ alkyl quaternary ammonium hydroxide intermediates. It has further been discovered that di $C_8$-$C_{12}$ alkyl quaternary ammonium carbonate quats are useful in wood preservative systems as they have improved leaching resistance, particularly without the use of the commonly used metal stabilizers or couplers, arsenic, chromium, copper, and zinc or combinations thereof.

The di $C_8$-$C_{12}$ alkyl carbonate quats prepared by the methods above, are useful as wood preservatives, as they have improved leaching resistance, particularly without the use of the commonly used, metal stabilizers or couplers, arsenic, chromium, copper, and zinc or combinations thereof.

Typically, quaternary ammonium compounds migrate or leach from wood under wet conditions, however. Common waterproofing compositions have not proven compatible with the quaternary ammonium compounds typically used in the industry, and therefore, they are not commonly used to hinder the leaching of these quats.

Typical waterproofers are waxes, lower molecular weight polyolefins, or dispersions or solutions thereof in hydrocarbon solvents. However, quaternary compositions, including those useful in the present invention, typically are water soluble. Generally, they are not soluble in these typical waterproofer solvent systems and are not compatible

with emulsified or dispersed waterproofers.

It has now been discovered that $C_1$-$C_{20}$ alkyl or aryl-substituted alkyl, $C_8$-$C_{20}$ alkyl, and particularly di $C_1$-$C_{12}$ alkyl, quaternary ammonium carbonates prepared by the methods described herein, are compatible with newly discovered polyhydroxyl or polyetherhydroxyl esters of fatty acids or polyether hydroxide waterproofers. Waterproofing and wood preservative systems prepared from the quats described herein exhibit enhanced resistance to leaching and meet waterproofing standards for heavy duty, ground, or millwork applications.

E. Müller (ED): "Methoden der organischen Chemie (Houben-Weyl), Vol. XI/2. Stickstoffverbindungen II und III", STUTTGART, G. THIEME VERLAG, PAGE(S) 620-626, in particular page 624, paragraph 3 - page 626, paragraph 1, discloses preparation of quaternary ammonium chlorides with a metal hydroxide in alcoholic solution.

US 5354434 discloses reaction of a quaternary ammonium hydroxide with carbon dioxide to form a quatenary ammonium carbonate.

## BRIEF DESCRIPTION OF THE DRAWINGS

Figure 1A is a graphic comparison of leaching of a wood preservative system according to the present invention and a wood preservative system of the prior art.

Figure 1B is an enlarged segment of the graph of Figure 1A.

## SUMMARY OF THE INVENTION

According to the present invention there is provided a method for the preparation of a $C_1$-$C_{20}$ alkyl or aryl-substituted alkyl, $C_8$-$C_{20}$ alkyl quaternary ammonium carbonate having the formula

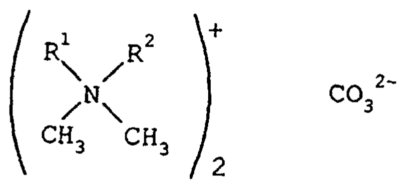

wherein $R^1$ is a $C_1$-$C_{20}$ alkyl or aryl-substituted alkyl group, and $R^2$ is a $C_8$-$C_{20}$ alkyl group, said method comprising

(a) reacting a $C_1$-$C_{20}$ alkyl or aryl-substituted alkyl, $C_8$-$C_{20}$ alkyl quaternary ammonium chloride reactant and a metal hydroxide reactant in a solvent comprising a $C_1$-$C_4$ normal alcohol, said metal hydroxide being present in an amount sufficient to yield a $C_1$-$C_{20}$ alkyl or aryl-substituted alkyl, $C_8$-$C_{20}$ alkyl quaternary ammonium hydroxide, a metal chloride, and optionally unreacted metal hydroxide; and

(b) reacting said $C_1$-$C_{20}$ alkyl or aryl-substituted alkyl, $C_8$-$C_{20}$ alkyl quaternary ammonium hydroxide and any unreacted metal hydroxide with carbon dioxide to yield said $C_1$-$C_{20}$ alkyl or aryl-substituted alkyl, $C_8$-$C_{20}$ alkyl quaternary ammonium carbonate and a metal carbonate.

Quaternary ammonium carbonates having the formula

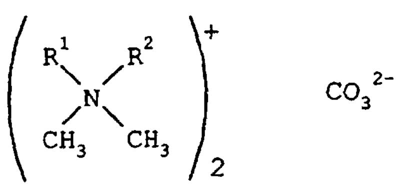

(IX)

wherein $R^1$ is a $C_1$-$C_{20}$ alkyl or aryl substituted alkyl group and $R^2$ is a $C_8$-$C_{20}$ alkyl group, and preferably wherein $R^1$ is the same as $R^2$ and $R^1$ is a $C_8$-$C_{12}$ alkyl group, are prepared by reacting two reactants, (a) $C_1$-$C_{20}$ alkyl or aryl-substituted alkyl, $C_8$-$C_{20}$ alkyl quaternary ammonium chloride and preferably a di $C_8$-$C_{12}$ alkyl quaternary ammonium chloride and (b) a metal hydroxide, in a solvent comprising a $C_1$-$C_4$ normal alcohol. The amount of metal hydroxide reactant is that amount sufficient to yield the corresponding $C_1$-$C_{20}$ alkyl or aryl-substituted, $C_8$-$C_{20}$ alkyl quaternary

ammonium hydroxide, and preferably the corresponding di $C_8$-$C_{12}$ alkyl quaternary ammonium hydroxide, a metal chloride, and optionally unreacted metal hydroxide. The resultant quaternary ammonium hydroxide and any unreacted metal hydroxide are then reacted with carbon dioxide to yield the corresponding quaternary ammonium carbonate.

Many $C_1$-$C_{20}$ alkyl or aryl-substituted alkyl, $C_8$-$C_{20}$ alkyl quaternary ammonium chlorides are suitable reactants, but di $C_8$-$C_{12}$ alkyl quaternary ammonium chloride is preferred, and didecyldimethylammonium chloride, and particularly, di-n-decyldimethylammonium chloride is most preferred. The selections of the $R^{12}$ and $R^{13}$ substituents of the chloride quat reactant are determinative of the hydroxy quat product.

Special mention is also made of processes wherein $R^{12}$ is a methyl, butyl, $C_8$ alkyl, $C_9$ isoalkyl, $C_{10}$ alkyl, $C_{12}$ alkyl, $C_{14}$ alkyl or benzyl group; and $R^{13}$ is a $C_{10}$ alkyl, $C_{12}$ alkyl, $C_{14}$ alkyl or $C_{16}$ alkyl group.

The metal hydroxide reactant is a mono-, bi-, or trivalent metal hydroxide, preferably a monovalent metal hydroxide, and most preferably an alkali metal hydroxide such as sodium hydroxide or potassium hydroxide. Special mention is made of potassium hydroxide. The metal chloride reaction product will precipitate and is easily removed, i. e. by filtration or the like, yielding a hydroxy quat/solvent reaction product. The hydroxy quat can be separated therefrom by drying or the like.

The reaction is conducted in a solvent which comprises a $C_1$-$C_4$ normal alcohol. Preferably, the solvent is ethanol, and most preferably, anhydrous ethanol.

The amount of metal hydroxide reactant typically is a stoichiometric amount with respect to the quaternary ammonium chloride reactant. Therefore, on a theoretical basis and if the reaction were complete and unequilibrated, there would be no excess of metal hydroxide reactant upon completion of the reaction. In practice, yield when using a stoichiometric amount of metal hydroxide reactant will range from about 65% to about 95%, but will vary, dependent in part upon the particular metal hydroxide reactant.

Yield can be furcher improved over conventional methods by utilization of a stoichiometric excess of metal hydroxide ranging from about 2% to about 20% excess. If an excess of metal hydroxide is used, yield will be increased to from about 95% to about 99%, again varying as above.

The unreacted metal hydroxide is soluble in the hydroxy quat/solvent mixture. Any excess or unreacted metal hydroxide is removed after the reaction is completed, and is preferably precipitated by subsequent reaction with carbon dioxide to yield the corresponding metal carbonate. The carbonate is insoluble in the hydroxy quat/solvent mixture and is easily removed, i.e. by filtration or the like. Alternatively, a solid metal bicarbonate, in which the metal corresponds to the metal of the metal hydroxide, can be added and slurried with the hydroxy quat/solvent mixture. The soluble metal hydroxide reacts with solid bicarbonate to yield the insoluble metal carbonate.The metal carbonate does not react further with the hydroxy quat.

Mixing, adding, and reacting of the components in the preparation of these hydroxy quats can be accomplished by conventional means known to those of ordinary skill in the art. The order of addition of reactants or solvent does not affect the process. Reactants and/or solvent can be added sequentially or simultaneously in any suitable reaction vessel.

Typically, the reactants and solvent will be stirred and heated to from about 20°C to about 70°C and held at that temperature for a period of from about 1 hour to about 5 hours. The reaction mixture is then cooled, first to room temperature and then to about 0°C where it is held for about 1 hour to about 2 hours. Any precipitated metal chloride is collected as is known in the art, i.e. such as by filtration.

Alternatively, the reactants and solvent can be stirred at a slightly elevated temperature, i.e. from about 20°C to about 40°C, to yield the hydroxy quat/solvent mixture. Hydroxy quat can be separated as above.

Quaternary Ammonium Carbonate

Although any quaternary ammonium carbonates are suitable for use in the present invention, preferred carbonate quats have the formula

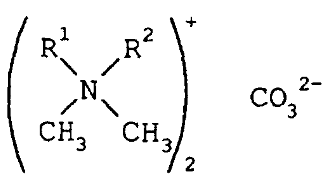

(XI)

wherein $R^1$ is a $C_1$-$C_{20}$ alkyl or aryl-substituted alkyl group, $R^2$ is a $C_8$-$C_{20}$ alkyl group, and preferably $R^1$ and $R^2$ are the same $C_8$-$C_{12}$ alkyl group.

Special mention is made of carbonate quats wherein $R^1$ is a methyl, $C_8$ alkyl, $C_9$ isoalkyl, $C_{10}$ alkyl, $C_{12}$ alkyl,

$C_{14}$ alkyl, $C_{16}$ alkyl, or benzyl group; and $R^2$ is a $C_{10}$ alkyl, $C_{12}$ alkyl, $C_{14}$ alkyl, or $C_{16}$ alkyl group.

Most preferred carbonate quats are didecyldimethylammonium carbonate wherein $R^1$ and $R^2$ are a $C_{10}$ alkyl group and preferably an n-$C_{10}$ alkyl group. Didecyldimethylammonium carbonate, when observed as a 70-80 percent by weight solution is a yellow/orange liquid that has a slightly fruity odor. This formulation has a flash point of about 160°F, and it reacts with carboxyl containing compounds.

One or more of these carbonate quats alone or in combination with the corresponding bicarbonate quat(s) and/or metal carbonate salt(s), preferably potassium carbonate salt, can be formulated in the present waterproofer, wood preservative systems.

The stability, and particularly the thermal stability, of carbonate quats is superior to that of hydroxy quats, making these carbonate quats suitable for concentrating and as stock intermediates for further processing.

One or more of these carbonate quats alone or in combination with the corresponding bicarbonate quat(s) and/or metal carbonate salt(s), preferably potassium carbonate salt, can be formulated as metal coupler-free wood preservative systems. These systems include biocidal effective amounts of at least one carbonate quat and a suitable solvent, including aqueous and non-aqueous solvents. Preferably, the solvent is an aqueous solvent including, but not limited to, water, aqueous alcohol such as aqueous ethanol, ammonia water, and the like, or a combination of any of the foregoing.

Although other conventional additives may be added as required for application to different substrates and for different uses as known to those of ordinary skill in the art, metal stabilizers are not required and, in fact, are not recommended to inhibit leaching of the quat from the substrate. Accordingly, wood substrates, such as lumber, timber, and the like, can be treated with metal coupler-free preservative systems which comprise the above carbonate quat (s) diluted in a suitable solvent as above.

The amount of di $C_8$-$C_{12}$ alkyl quaternary ammonium carbonate(s) used to treat the substrate'is a biocidal effective amount, i.e. that amount effective to inhibit the growth of or to kill one or more organism that causes wood rot, to inhibit sap stain, or a combination thereof. Such organisms include, but are not limited to, Trametes viride or Trametes versicolor, which cause a white rot; Gloeophyllum trabeum, which causes a brown rot; and Aspergillus niger, which causes sap stain/mold.

Typically, a wood preservative system will comprise from about 0.1 to about 5 parts by weight of the carbonate quat(s) and from about 95 to about 99.9 parts by weight of solvent based upon 100 parts by weight of quat and solvent combined. Most preferably, the wood preservative system of the present invention will comprise from about 1 to about 2 parts by weight of carbonate quat(s) and from about 98 to about 99 parts by weight of solvent on the same basis.

Treatment of the substrate is accomplished by any means known to those of ordinary skill in the art including, but not limited to, dipping, soaking, brushing, pressure treating, or the like. The length of treatment required will vary according to treatment conditions, the selection of which are known to those skilled in the art.

These metal coupler-free preservative systems display greater resistance to leaching than wood preservatives currently used in the industry. Resistance to leaching is defined as retention of a biocidal effective amount, and preferably at least about 2% by weight, of carbonate quat(s) in the substrate over a prolonged period of at least about 100. hours and preferably about 350 hours. Applicants hypothesize, without being bound by any theory, that the carbonate quat reacts or complexes with the woody matrix of the substrate, thereby "fixing" it in the substrate. It is also believed that the long chain carbonate quat(s) and the wood preservative systems that include such quats enhance waterproofing properties of treated substrates.

Although certain carbonate quats can be prepared by a variety of methods, applicants have discovered an indirect synthesis method that can be used to prepare a variety of $C_1$-$C_{20}$ alkyl or aryl-substituted alkyl, $C_8$-$C_{20}$ alkyl quaternary ammonium carbonate compounds, preferably di $C_8$-$C_{12}$ alkyl quaternary ammonium carbonate compounds, and most preferably didecyldimethylammonium carbonate, according to the following scheme

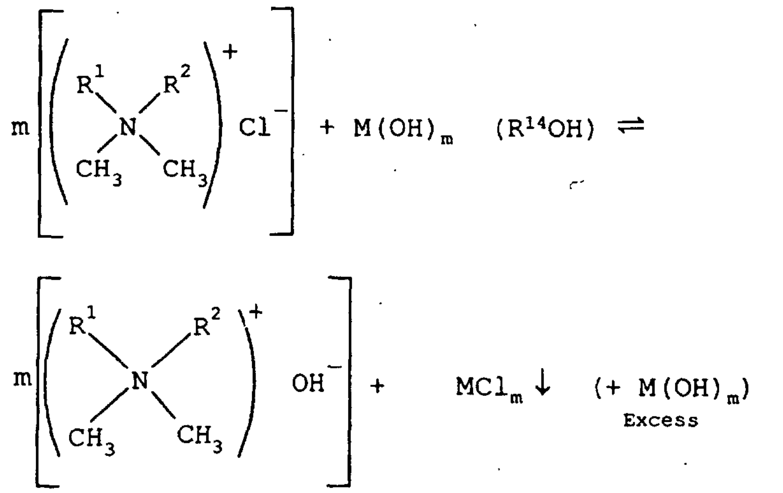


(XXVI)

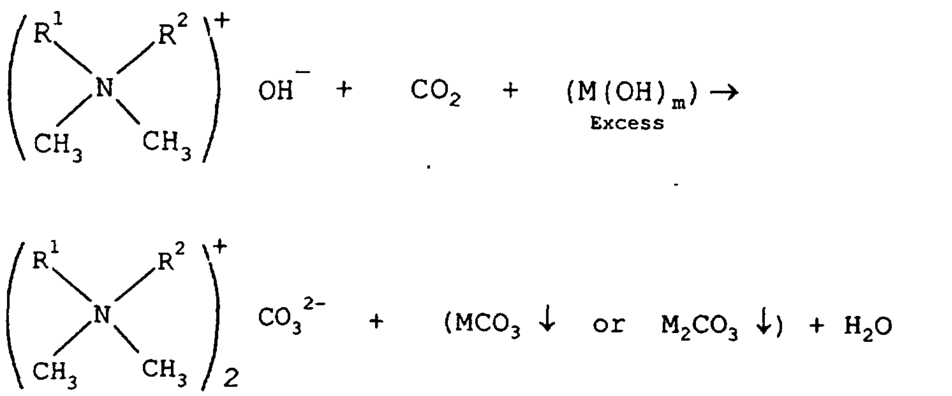


(XXVII)

wherein $R^1$ is a $C_1$-$C_{20}$ alkyl or aryl-substituted alkyl group; $R^2$ is a $C_8$-$C_{20}$ alkyl group; and preferably $R^1$ is the same as $R^2$ and $R^1$ is a $C_8$-$C_{12}$ alkyl group; $R^{14}$ is a straight chain $C_1$-$C_4$ alkyl group; M is a mono-, bi-, tri-valent metal, preferably a mono-valent metal, and most preferably an alkali metal; and m is 1 if M is mono-valent, 2 if M is di-valent, and 3 if M is trivalent.

A $C_1$-$C_{20}$ alkyl or aryl-substituted alkyl, $C_8$-$C_{20}$ alkyl, and preferably a di $C_8$-$C_{12}$ alkyl, quaternary ammonium chloride is used as a starting material and is reacted with a metal hydroxide to yield a $C_1$-$C_{20}$ alkyl or aryl-substituted alkyl, $C_8$-$C_{20}$ alkyl, and preferably a di $C_8$-$C_{12}$ alkyl, quaternary ammonium hydroxide intermediate. The hydroxy quat intermediate(s) and any excess metal hydroxide are then reacted with carbon dioxide to yield the carbonate quat(s) and the metal carbonate.

Many di $C_8$-$C_{12}$ alkyl quaternary ammonium chlorides are suitable reactants to prepare the intermediate hydroxy quat and are described above. The selections of the $R^1$ and $R^2$ substituents of the chloride quat reactant are determinative of the hydroxy quat intermediate, and therefore, of the carbonate quat product.

The metal hydroxide reactant is also as described above.

The metal chloride first step reaction product will precipitate and is easily removed, i.e. by filtration or the like, yielding a hydroxy quat/solvent reaction product. The hydroxy quat can be separated therefrom by drying or the like, if desired.

The first reaction (XXVI) is conducted in a solvent as described above, and the amount of metal hydroxide reactant is as described above.

Hydroxy quat and any unreacted metal hydroxide are then reacted with at least a stoichiometric equivalent of carbon dioxide to yield the quaternary ammonium carbonate(s), and if any unreacted metal hydroxide is present, the metal carbonate(s). The conversion of the metal hydroxide to the metal carbonate is the preferred reaction of the two carbonations and will proceed more rapidly. The metal carbonate will precipitate and can be separated easily, i.e. by filtration or the like, leaving the stable carbonate quat(s) or carbonate quat(s)/solvent reaction product.

The carbonation step can also produce the bicarbonate quat or the metal carbonate quat as byproducts. The

carbonate quat alone or in combination with the bicarbonate quat and/or the metal carbonate quat are suitable for use in the metal coupler-free wood preservative systems of the present invention. These carbonate quats or carbonate/bicarbonate/metal carbonate compositions, do not require a metal coupler for stabilization in a wood substrate. Completely metal-free wood preservative systems are preferred. However, if a metal carbonate quat is included in the system, preferably the metal is not a metal currently used as a coupler, and most preferably, it is an alkali metal and does not pose environmental or corrosion hazards or concerns.

Mixing, adding, and reacting of the components in the preparation of these carbonate quats can be accomplished by conventional means known to those of ordinary skill in the art. The order of addition of reactants or solvent in any individual step does not affect the process. Reactants and/or solvent can be added sequentially or simultaneously in any suitable reaction vessel. For example, the metal hydroxide may be dissolved in alcohol and the resultant mixture added to the chloride quat or the chloride quat may be dissolved in alcohol and the metal hydroxide added to the resultant mixture.

The carbon dioxide is generally bubbled for a suitable period known to those of ordinary skill in the art through the hydroxy quat/solvent supernatant after the metal chloride precipitate has been separated. Alternatively, the carbon dioxide can be added as solid dry ice directly to the hydroxy quat. Typically, this time varies from about 0.5 hour to about 1 hour at ambient temperature. Any precipitated metal carbonate is collected as is known in the art, i.e., such as by filtration.

DESCRIPTION OF THE PREFERRED EMBODIMENTS

The following examples are illustrations of the claimed invention. All parts and percentages are given by weight unless otherwise indicated.

Quaternary compounds are quantified by two phase titration with sodium laurylsulfate and an indicator. The mixture is buffered to a pH of 10. Swell index is calculated as

Swell index is calculated as

$$\left( \frac{\text{(Swell of Control - Swell of Sample)}}{\text{Swell of Control}} \times 10 \right) \times 100$$

Preparation of Carbonate Quats

Example 1

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered to remove 7.2 g of potassium carbonate (6.2 g calculated), and the filtrate was concentrated to yield an orange/brown liquid with 80-85% carbonate quat in water/ethanol and less than 0.1% chloride quat having a product with 98 to 99% exchanged quat purity.

Example 2

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was heated to 50°C and stirred for one hour.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50

g of carbon dioxide (from sublimed carbon dioxide) were bubbled over one half hour. The resultant mixture was then filtered, and the filtrate was concentrated to yield an orange/brown liquid. Yield was similar to that of Example 1.

Treatment of Wood Substrates

Example 3

End grain pine wafers were weighed and then soaked with didecyldimethylammonium carbonate until a weight gain of 30% was observed.

The treated wafers were then placed in water and weighed periodically to determine resistance to leaching.

Results are illustrated in Figures 1A and 1B.

Comparative Example 3A

The procedure of Example 3 was followed substituting didecyldimethylammonium chloride for the didecyldimethylammonium carbonate.

Results are illustrated in Figures 1A and 1B.

Figures 1A and 1B illustrate that the carbonate quat resists leaching for extended periods while the chloride quat leaches to levels of 1% or less in a relatively short period.

Examples 4 and 5 and Comparative Examples 4A, 4B, 5A and 5B

A 25.4×1.27×1.91 $cm^3$ (10"×0.5"×0.75") piece of ponderosa pine was equilibrated, weighed, and heated for two hours at 60°C. The wood was treated with a treating solution of 2% didecyldimethylammonium carbonate in water solvent by heating in the solution at 60°C to 80°C for one hour, cooling and standing overnight, and then being subjected to a second warm to cool cycle. The samples were allowed to dry to constant weight, and the uptake was determined by comparing starting and finishing weights.

The samples were then heated for two hours at 60°C, and the weight of the warm treated samples was compared to the oven dried sticks before treatment.

Additional examples were prepared either omitting the carbonate quat, substituting a chloride quat, or using 1% quat in a 3% aqueous ammonia solvent.

Formulations and results are illustrated in Table 1.

TABLE 1

| Weight Uptake from Quat Solutions | | | | | | |
|---|---|---|---|---|---|---|
| Example | 4 | 4A | 4B | 5 | 5A | 5B |
| Solvent | Water | Water | Water | 3% Ammonia | 3% Ammonia | 3% Ammonia |
| Quat | Carbonate | - | Chloride | Carbonate | - | Chloride |
| Weight Uptake (%) | 1.8 | -0.4 | 0.6 | 1.6 | -0.6 | 2.0 |

Examples 4 and 5, when compared with Comparative Examples 4A, 4B, 5A, and 5B respectively, illustrate the ability of the carbonate quats of the present invention to be applied to wood substrates. The carbonate quat is absorbed better than the chloride quat in water, and is absorbed similarly to the art accepted chloride quat in ammonia/water. However, the carbonate quats can be used without metal coupling agents in treating wood substrates.

Examples 6 . 9 and Comparative Examples 6A, 6B, 9A and 9B

A piece of wood was treated according to the procedure of Example 4. The piece of wood was then soaked in water at room temperature for 24 hours, dried to constant weight, and weighed to determine how much chemical remained. The piece of wood was soaked for 96 additional hours (120 hours total), dried to constant weight, and weighed to determine the leaching of quat from the treated wood. The water was changed several times during this period.

Additional examples were prepared with different quat concentrations, different anions, and different solvents.

Formulations and results are illustrated in Table 2.

TABLE 2

Leaching of Quat

| Example | 6 | 6A | 6B | 7 | 8 | 9 | 9A | 9B |
|---|---|---|---|---|---|---|---|---|
| Solvent | Water | Water | Water | Water | Water | 3% Ammonia | 3% Ammonia | 3% Ammonia |
| Quat | 2% Carbonate | | 2% Chloride | 2.5% Carbonate | 5% Carbonate | 2% Carbonate | | 2% Chloride |
| Weight Uptake (%) | 1.8 | 0.4 | 0.6 | 1.1 | 1.8 | 1.6 | 0.6 | 2 |
| Retained Quat at 24 Hours (Absolute %/ Relative %) | 2/110 | -0.2/- | 0.5/83 | -/100+ | -/100 | 1.7/100 | -0.3/- | 1.7/85 |
| Retained Quat at 120 Hours (Absolute %/ Relative %) | 1.6/80 | -0.2/- | 0.4/67 | -/- | -/- | 1.2/75 | -0.3/- | 1.36/65 |

Examples 6-9 and particularly Example 2, when compared with Comparative Examples 6A and 6B, and Example 9, when compared with Comparative Examples 9A and 9B, demonstrate the improved retention properties of

carbonate quats over conventional chloride quats, particularly in the absence of metal stabilizers.

## Claims

**1.** A method for the preparation of a quaternary ammonium carbonate having the formula

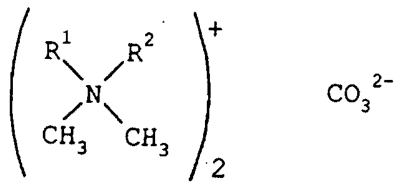

wherein $R^1$ is a $C_1$ - $C_{20}$ alkyl or aryl-substituted alkyl group, and $R^2$ is a $C_8$ - $C_{20}$ alkyl group, said method comprising

(a) reacting a $C_1$ - $C_{20}$ alkyl or aryl-substituted alkyl, $C_8$ - $C_{20}$ alkyl quaternary ammonium chloride reactant and a metal hydroxide reactant in a solvent comprising a $C_1$ - $C_4$ normal alcohol, said metal hydroxide being present in an amount sufficient to yield a $C_1$ $C_{20}$ alkyl or aryl-substituted alkyl, $C_8$-$C_{20}$ alkyl quaternary ammonium hydroxide, a metal chloride, and optionally unreacted metal hydroxide; and
(b) reacting said $C_1$ - $C_{20}$ alkyl or aryl-substituted alkyl, $C_8$ - $C_{20}$ alkyl quaternary ammonium hydroxide and any unreacted metal hydroxide with carbon dioxide to yield said quaternary ammonium carbonate and a metal carbonate.

**2.** A method as defined in claim 1, wherein $R^1$ in the quaternary ammonium carbonate is the same as $R^2$ and $R^1$ is a $C_8$-$C_{12}$ alkyl group.

**3.** A method as defined in claim 2, wherein $R^1$ is a $C_{10}$ alkyl group.

## Patentansprüche

**1.** Verfahren zur Herstellung eines quartären Ammoniumcarbonats der Formel

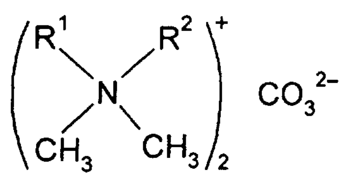

worin $R^1$ eine $C_1$-$C_{20}$-Alkyl- oder arylsubstituierte Alkylgruppe und $R^2$ eine $C_8$-$C_{20}$-Alkylgruppe ist, umfassend

(a) die Umsetzung eines quartären $C_1$-$C_{20}$-Alkyl- oder arylsubstituierten Alkyl-$C_8$-$C_{20}$-alkylammoniumchlorid-Reaktanten und eines Metallhydroxidreaktanten in einem Lösungsmittel, welches einen normalen $C_1$-$C_4$-Alkohol umfasst, wobei das Metallhydroxid in einer Menge vorhanden ist, die ausreicht um ein quartäres $C_1$-$C_{20}$-Alkyl- oder arylsubstituiertes Alkyl-$C_8$-$C_{20}$-alkylammoniumhydroxid, ein Metallchlorid und gegebenenfalls unumgesetztes Metallhydroxid zu bilden, und
(b) die Umsetzung des quartären $C_1$-$C_{20}$-Alkyl- oder arylsubstituierten Alkyl-$C_8$-$C_{20}$-alkylammoniumhydroxids und jeglichen unumgesetzten Metallhydroxids mit Kohlendioxid zu dem quartären Ammoniumcarbonat und einem Metallcarbonat.

**2.** Verfahren nach Anspruch 1, worin $R^1$ im quartären Ammoniumcarbonat gleich wie $R^2$ und $R^1$ eine $C_8$-$C_{12}$-Alkylgruppe ist.

**3.** Verfahren nach Anspruch 2, worin $R^1$ eine $C_{10}$-Alkylgruppe ist.

**Revendications**

1. Procédé de préparation d'un carbonate d'ammonium quaternaire ayant la formule

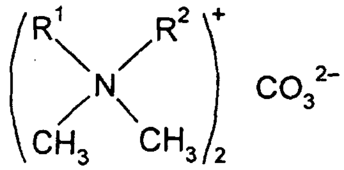

dans laquelle $R^1$ est un groupement alkyle en $C_1$-$C_{20}$ ou alkyle en $C_1$-$C_{20}$ substitué par un aryle et $R^2$ est un groupement alkyle en $C_8$-$C_{20}$, ledit procédé comprenant

(a) la réaction d'un chlorure d'ammonium quaternaire d'alkyle en $C_1$-$C_{20}$ ou d'alkyle en $C_1$-$C_{20}$ substitué par un aryle et d'alkyle en $C_8$-$C_{20}$ et d'un hydroxyde métallique dans un solvant comprenant un alcool normal en $C_1$-$C_4$, ledit hydroxyde métallique étant présent dans une quantité suffisante pour donner un hydroxyde d'ammonium quaternaire d'alkyle en $C_1$-$C_{20}$ ou d'alkyle en $C_1$-$C_{20}$ substitué par un aryle et d'alkyle en $C_8$-$C_{20}$, un chlorure métallique et facultativement l'hydroxyde métallique qui n'a pas réagi ; et
(b) la réaction dudit hydroxyde d'ammonium quaternaire d'alkyle en $C_1$-$C_{20}$ ou d'alkyle en $C_1$-$C_{20}$ substitué par un aryle et d'alkyle en $C_8$-$C_{20}$ et de tout hydroxyde métallique qui n'a pas réagi avec du dioxyde de carbone pour donner ledit carbonate d'ammonium quaternaire et un carbonate métallique.

2. Procédé selon la revendication 1, dans lequel $R^1$ dans le carbonate d'ammonium quaternaire est le même groupement que $R^2$ et $R^1$ est un groupement alkyle en $C_8$-$C_{12}$.

3. Procédé selon la revendication 2, dans lequel $R^1$ est un groupement alkyle en $C_{10}$.

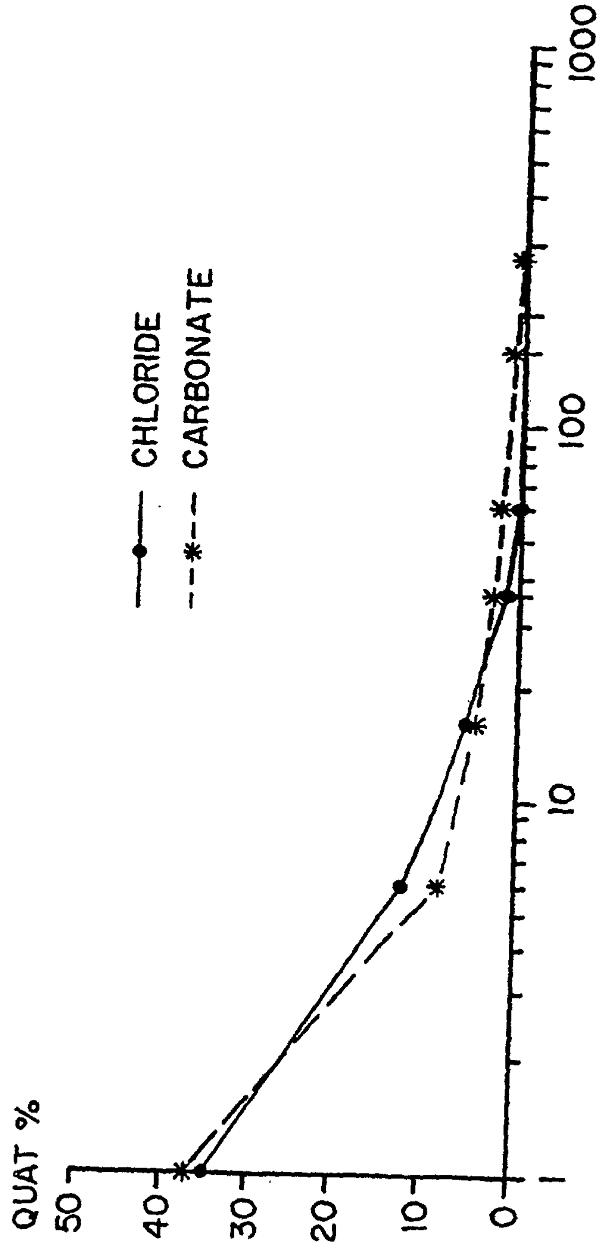
QUAT %
50
40
30
20
10
0
1
10
100
1000
TIME OF LEACHING (HOURS)
CHLORIDE
CARBONATE

FIG. 1A

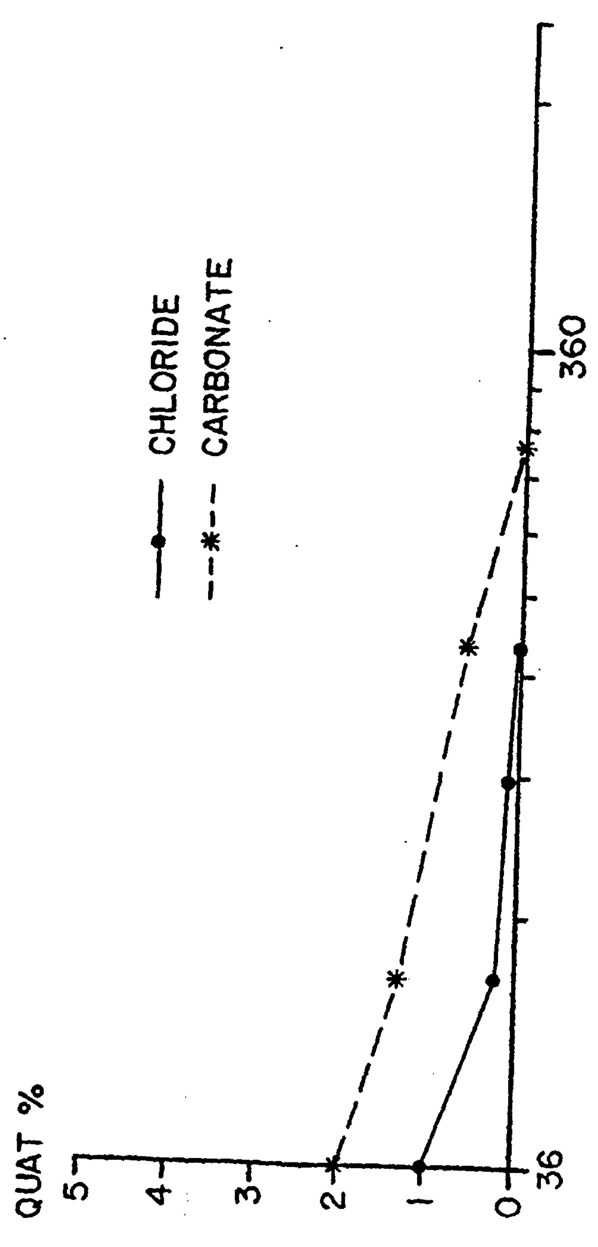
QUAT %
5
4
3
2
1
0
36
360
TIME OF LEACHING (HOURS)
CHLORIDE
CARBONATE

FIG. 1B